(19) **Europäisches Patentamt European Patent Office Office européen des brevets**

(11) **EP 2 451 358 B1**

(12) **EUROPEAN PATENT SPECIFICATION**

(45) Date of publication and mention
of the grant of the patent:
**09.08.2017 Bulletin 2017/32**

(51) Int Cl.:
*A61B 6/00* *(2006.01)* *A61B 8/00* *(2006.01)*
*A61B 5/06* *(2006.01)* *A61B 1/00* *(2006.01)*
*A61B 90/00* *(2016.01)* *A61B 34/00* *(2016.01)*
*A61B 6/12* *(2006.01)*

(21) Application number: **10740304.0**

(22) Date of filing: **29.06.2010**

(86) International application number:
**PCT/IB2010/052955**

(87) International publication number:
**WO 2011/004288 (13.01.2011 Gazette 2011/02)**

(54) **VISUALIZATION OF PHYSIOLOGICAL PARAMETERS**

VISUALISIERUNG VON PHYSIOLOGISCHEN PARAMETERN

VISUALISATION DE PARAMÈTRES PHYSIOLOGIQUES

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **06.07.2009 EP 09164628**

(43) Date of publication of application:
**16.05.2012 Bulletin 2012/20**

(73) Proprietor: **Koninklijke Philips N.V.**
**5656 AE Eindhoven (NL)**

(72) Inventors:
• **NACHABÉ, Rami**
**5656 AE Eindhoven (NL)**
• **HENDRIKS, Bernardus, Hendrikus, Wilhelmus**
**5656 AE Eindhoven (NL)**
• **BABIC, Drazenko**
**5656 AE Eindhoven (NL)**
• **BRAUN, Augustinus, Laurentius**
**5656 AE Eindhoven (NL)**

• **VAN DER VOORT, Marjolein**
**5656 AE Eindhoven (NL)**
• **DESJARDINS, Adrien, Emmanuel**
**5656 AE Eindhoven (NL)**
• **MIHAJLOVIC, Nenad**
**5656 AE Eindhoven (NL)**
• **HARBERS, Rik**
**5656 AE Eindhoven (NL)**

(74) Representative: **van Velzen, Maaike Mathilde**
**Philips Intellectual Property & Standards**
**High Tech Campus 5**
**5656 AE Eindhoven (NL)**

(56) References cited:
EP-A1- 1 690 491     EP-A2- 1 415 608
WO-A1-2005/013841     WO-A2-02/064011
US-A- 6 016 439     US-A1- 2007 016 028
US-A1- 2008 283 771     US-B1- 6 564 087

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The invention relates to a medical imaging apparatus for providing information about an object. The invention further relates to a method for providing information about an object.

BACKGROUND OF THE INVENTION

Information about an object can be required, for example, for the diagnostics of diseases or during an intervention, such as a surgical intervention, to provide necessary or desired knowledge of the object, for example, in order to be able to perform further interventional steps. This knowledge can serve as a kind of feedback, for example in cases where the results of the intervention are difficult to detect, such as in cases where the object of interest is a patient, where medical intervention can be performed in such a way that the actual interventional process or even the result cannot be detected by the person performing the intervention. Medical imaging is therefore used in different applications. For example, the data these images are based upon can be derived by means of X-rays, MRI, Ultrasound, SPECT etc. Especially in minimally invasive procedures, the surgeon may thus be provided with the information necessary for performing the intervention. Furthermore, conventional X-ray imaging is used in endovascular interventional procedures as well as in percutaneous interventions. In order to provide soft tissue information, the so called soft tissue imaging, known as XperCT, is used. Further, percutaneous needle mediated interventions are widely used in a large variety of diseases where taking biopsies of tissue is used for tissue examination. For the definition of the needle trajectory as well as for image guided needle tracking, imaging modalities, such as X-ray, CT, MR etc. are used. With a needle integrated with optical fibers such as a photonic needle, it is possible to acquire optical spectroscopy data. Hence, the user is provided with two separate sources of information, for example X-ray images and optical information, displayed at two different places such as two monitors. For example, US 2007/0016028 A1 describes the use of several monitors of which one is a so-called navigation monitor displaying real time images in one window and real time EP signals in a further window. But it has been shown that the user, for example a physician, has difficulties conceiving all of the provided information. Especially during medical procedures that conventionally use X-ray information only, the presentation of additional information may hamper the normal work flow. Further, it has also been shown that due to an increasing number of parameters that can be displayed to provide information about the object of interest, for example the patient, the user sometimes experiences a fatigue that leads to a decrease in the user's performance and concentration during the intervention.

EP 1 415 608 A2 relates to real-time monitoring and mapping of ablation lesion formation in the heart, and describes that a parameter is sensed that is indicative of a level of ablation at a respective site and that, during the ablation procedure. A map of the heart is displayed and indications of the respective levels of ablation are designated at the respective sites. For this purpose, a three-dimensional reconstruction of the ablation lesion is generated and displayed. The map of the heart is described as a geometric and electrical map of the cardiac chambers acquired prior to the beginning of the ablation procedure. During the ablation procedure, the reconstruction of the ablation lesion is overlaid on the pre-acquired cardiac map.

SUMMARY OF THE INVENTION

Hence, there may be a need to improve the preparation and the visual reception of information relating to an object during medical interventions.

The object is reached with a medical imaging apparatus and a method, according to the independent claims.

According to an exemplary embodiment of the information, a medical imaging apparatus for providing information about an object is provided that comprises an image acquisition device, an interventional device, a processing device and a display device. The image acquisition device is adapted to detect object data from at least one region of interest of an object and to provide the object data to a processing device. The image acquisition device comprises an X-ray imaging system or a computer tomography system or a magnetic resonance imaging modality or an acoustic imaging system using ultrasound waves. The interventional device is adapted to detect physiological parameters of the object depending on the position of a predetermined area of the interventional device in relation to the object and to provide the physiological parameters to the processing device. The processing device is adapted to transform at least a part of the object data into image data and to convert the physiological parameters into physiological data. The processing device is further adapted to modify at least one image parameter of the image data depending on the physiological data and to thereby transform the image data into modified live image data. The processing device is adapted to provide the modified live image data to the display device. The display device is adapted to display the modified live image.

The modification may comprise changing such parameters as contrast or brightness as well as color or saturation etc. The modification can be performed for the whole image data or for only selected areas or parts. The selection may be predetermined by the user and can relate to certain areas of the image and/or certain content of the image data which thus may be analyzed concerning the shown content.

The term "depending" means that a change in the physiological data, for example due to a movement of the interventional device or due to a change in the object's condition, leads to a change in the modified live image being displayed. The dependency may be defined as being directly coupled. The dependency may as well be ruled by a predetermined equation applied to the results of the physiological data detection.

This provides the advantage that the user, for example the physician, can proceed the intervention obtaining the additional information in a way that is not compromising the time he or she can spend on interpreting the image. In other words, the user is provided with additional information without requiring additional attention or influence on the concentration because the user only has to pay attention to one image instead of several images. A further advantage lies in the fact that by modifying the image data a loss of image content can be prevented. Simply said, the image data, in other words the image displayed, is not affected in a way that content is cut out or covered for example, by displaying the additional information.

Another advantage of the invention may be that additional information in form of the physiological data, can be provided to the user without the need of an extra display unit or separate window within a single display. In other words, the image data containing, for example, conventional information about the object, is not affected by the provision of the additional physiological data. By providing the additional data in form of the modified image the user can concentrate on the image provided on the display so that the reception of the content displayed is not interfered by the provision of the additional data. Thus, the additional information can be perceived by the user in a way which does not lead to fatigue or a decrease in concentration.

Since the additional information by the modified image relates to the object data derived by the interventional device, an interaction of the user in form of a change in the position of the device has the effect that the modified image is changing accordingly because the extraction of the physiological data and the modification is performed in real time, or at least in near real time.

Of course, the appearance of the additional information can be coded depending on the medical imaging methods used and according to the type of intervention applied to ensure a widespread use of the invention and to allow an easy perception of the information communicated to the user. For example, the selection of the type of additional data and its color or brightness or pattern should be performed depending on the physicians' needs.

One of the advantages of the invention is that the user can view the image data focusing on the content displayed on the display unit. Thus, the user can fully concentrate on the information shown on the display.

By providing the modified image to the user, the user is supported so that the process of making guidance decisions, which have to be made extremely quickly in order to avoid tissue damage, is relieved because the diffuse reflectance spectroscopy (DRS) information is presented in a concise and unobtrusive way that does not distract the physician too much from the original intervention.

But the present invention also provides the use of the potential perception or cognition that is so far unused. In the peripheral sub-zone of the user's area of view it is, for example, usually not possible to read or detect numbers or values but it is still possible to detect changes of brightness or color or patterns in this peripheral part of the field of view. Hence, in case the additional information is displayed by modifying the peripheral zone of the image, information or aspects can be communicated to the user by rather simple visual effects, for example a change of color or change of brightness representing determined data. In other words, the physiological data may be displayed in a rather simple way.

In an exemplary embodiment, a user interface to input data is provided in order to allow a determination of the kind of indicative data according to the user's needs.

For example, the physiological parameters may be an anatomical parameter.

In an exemplary embodiment, the physiological parameters are represented by optical information. For example, the optical information relates to color coding according to the field of intervention or examination respectively.

In a further exemplary embodiment, for certain conditions of the patient, thresholds can be pre-determined and monitored by the interventional device. Once a parameter reaches a critical value, that means once a threshold is reached, the modified image provides respective graphical information to the user.

In an exemplary embodiment, physiological parameters may be detected by the interventional device that are not provided by the detected object data.

This provides the advantage that the object data can be detected with a larger scale or lower resolution, whereas the interventional device provides a smaller scale that means a higher resolution or higher grade of details providing additional knowledge of the actual situation to the user.

For example, the apparatus may be adapted to determine the position of the interventional device depending on the physiological parameters.

For example, in case a certain physiological parameter is known from previous examination, the location of the interventional device in relation to the predetermined feature can be achieved by the physiological data.

For example, the apparatus is adapted to visualize the interventional device, for example in cases, where the interventional device is not visible in the detected object data, such as a glass needle under fluoroscopy. By obtaining information about the tissue at the needle's distal end, for example, it can be derived that the needle is in a certain type of tissue. Since certain tissue parameters can be identified for certain spatial relation to the structure visible on the fluoroscopy image, it is possible to know where or in which type of tissue the needle is at the moment.

The image acquisition device is an imaging modality such as an X-ray imaging system, a computer tomography system (CT), magnetic resonance imaging modality (MR), ultrasound (US) or the like.

For example, the image acquisition device may be an X-ray apparatus acquiring fluoroscopic image.

According to another exemplary embodiment, the image data comprises a background and at least one parameter of at least a part of the background is modified in dependency of the physiological data.

For example, the background corresponds to the optical information representing the physiological parameter. Of course, several physiological parameters can be represented in such a way.

In an exemplary embodiment, the central part of the display comprises an image provided by the image acquisition device, such as an X-ray image with a background, for example a surrounding background, and the background changes in real time at each different position of the interventional device while the central part of the image is not effected. For example, the central part can have a circular form, whereas the background may be the rest of a square display.

According to another exemplary embodiment, the interventional device is shown in the modified live image with its trajectory and a graphical representation of the physiological data is shown along the trajectory of the interventional device.

For example, the graphical representation may be a colored marker, such as colored circles, for example with varying diameters to indicate intensity or other aspects of the physiological parameter. By providing the graphical representation along the trajectory of the interventional device, the user is provided with further information, namely the path within which the device has already been moved along. This may also indicate the direction for further movement of the interventional device.

According to another exemplary embodiment, the interventional device is shown in a modified live image and the color of the interventional device is modified depending on the detected physiological data.

This provides the advantage that the user is provided with additional information without meaning a cutout or covering of image information.

For example, the interventional device is arranged to provide molecular information. For example, during a needle intervention the molecular information can inform the user about the molecular condition at the tip of the needle inserted by the user.

In another exemplary embodiment, the physiological data is obtained by non-optical methods.

In a further exemplary embodiment, the data may be based on measuring temperature and/or pH to provide additional information to the morphology data.

For example, measuring temperature or pH can be useful to detect whether a site is reached where tissue is inflamed.

For example, the interventional device may be a biopsy needle, a canula, a trocar, a catheter or an endoscope.

Thus, the present invention is suitable for different applications or examination procedures respectively and can therefore be used in a wide range of different interventions.

According to a further exemplary embodiment, the interventional device is an optical needle with at least one integrated optical fiber adapted to acquire an optical spectrum to be translated into the physiological parameters.

For example, the translation may be achieved by spectroscopy.

In an exemplary embodiment, optical and non-optical methods are used in combination. This enables to provide sophisticated information to the surgeon for example.

According to a further exemplary embodiment, the interventional device is adapted to detect physiological parameters of the object, depending on the position of at least two different predetermined areas of the interventional device in relation to the object.

For example, the interventional device may be an optical needle and the needle may comprise more than two fibers such that spatial differences in the tissue to the left and right side, for instance, are detectable. This may be visualized on the display by different colors in the left and/or right part of the displayed image. One of the advantages is that a user is provided with differentiated information leading to an even better understanding of the situation within the object of interest.

According to a further exemplary embodiment, the contribution of blood, fat and water in the tissue at the predetermined location of the interventional device is converted into a color code and the color code is used for the modification of the image data.

Instead of or in addition to the parameters mentioned above, other parameters can be used for color coding as well.

The color code may be an RGB number, for example, blood may be represented by the color red, fat by the color green and water by the color blue.

For example, if the tissue content is measured on a scale from 0 to 1 for each of the selected parameters, this information can be converted into RGB numbers, using the following formula where R;G;B correspond respectively to red, green and blue (on a scale of 0 to 1):

$$R = ([blood]) / ([blood] + [water] + [fat])$$

$$G = ([fat]) / ([blood] + [water] + [fat])$$

$$B = ([\text{water}]) / ([\text{blood}] + [\text{water}] + [\text{fat}])$$

where [X] corresponds to the contribution value of element X = {blood; fat; water}.

For example, at a particular position, the contribution of blood, water and fat is respectively 0.8; 0.1; and 0.2. To convert this information into RGB numbers, the above-mentioned formula may be used. Thus, the RGB number is RGB = (0.8; 0.2; 0.1) which is a color close to red. In other words, the background 312 is displayed in a red color.

According to a further exemplary embodiment, the processing device is adapted to analyze the physiological data such that the approach of an important structure is determined and the image date is modified such that graphical instructional information is provided to the user.

For example, a first color may be used to indicate that the interventional device is not in danger of approaching the important structure, the color green for example. A second color may be used to indicate that the user must be careful in approaching the important structure, for example the color yellow. A third color may be used to indicate that the interventional device is very close to the important structure, for example by the color red. The important structure can be, for example, a vessel which the user does not want to perforate.

In another exemplary embodiment, different colors may correspond to different distances between, for example, chromophores and the probe, i.e. the needle directed by the user. For example, a first color could correspond to large distances when the probe is far from the target tissue and a second color could correspond to the case where the probe is within the target tissue.

According to a further exemplary embodiment, the interventional device may be adapted to acquire microscopic image data of the tissue in front of the needle, to detect the physiological data from the microscopic image data and the image data is modified with at least a part of the microscopic image data.

The acquisition may be performed, for example, by using a scanning fiber confocal scanner.

In an exemplary embodiment, the microscopic image data may be integrated into the modified image.

In a further exemplary embodiment, a combination of a photonic needle with X-ray provides the advantage that it is possible to combine macro-scale morphological information with micro-scale physiological or microscopic information. Hence, the image data can also be modified depending on the microscopic image.

For example, an interface unit is provided and adapted to control the display of the microscopic image data. This provides the advantage that the additional microscopic image data is only present in the image when the user actually wants to refer to the microscopic image data. Of course, the image displayed comprises the physiological parameters because the image has been modified.

The user interface may be, for example, a pedal, a mouse, a touch screen, a voice control device or the like adapted to enter a command.

In a further exemplary embodiment, the imaging apparatus comprises an X-ray imaging system with an X-ray image acquisition device comprising a source of X-ray radiation and an X-ray image detection module located opposite the source of X-ray radiation and a device provided to receive an object to be examined. Further, an interface unit is arranged to input information by the user. A control unit is connected to both the detection module and the radiation source and the interface unit as well as to the interventional device.

In another exemplary embodiment, the examination arrangement comprises an acoustic imaging system using ultrasound waves to generate images.

In a further exemplary embodiment, the examination arrangement comprises an MRI arrangement.

In a further exemplary embodiment, the examination arrangement comprises a SPECT arrangement.

According to a further aspect of the invention, a method for providing information about an object is provided according to independent claim 10. For example, the image data may comprise a background, for example, surrounding a central part, and the step of modifying may include a step of modifying at least one parameter of at least a part of the background depending of the second object data.

Further, the interventional device may be an optical needle and the physiological data may be detected in form of optical data. The background may have a color that corresponds to the optical data.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

However, the computer program may also be presented over a network like the world wide web and can be down loaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

BRIEF DESCRIPTION OF THE DRAWINGS

The aspect defined above and further aspects, features and advantages of the present invention can also be derived from the examples of embodiments to be described herein after and are explained with reference to examples of embodiments, but to which the invention is not limited. The invention will be described in more detail hereinafter with reference to the drawings.

Fig. 1 schematically shows a medical imaging apparatus according to the invention;
Fig. 2 shows a flow chart of a method for providing information about an object according to the invention;
Fig. 3 shows a contribution of different parameters for different positions of an interventional device;
Fig. 4 schematically shows an exemplary embodiment according to the invention of a modified live image;
Fig. 5 schematically shows a further exemplary embodiment of a modified live image;
Fig. 6 schematically shows a further exemplary embodiment of a modified live image;

Fig. 7 schematically shows a further exemplary embodiment of a modified live image;
Fig. 8 schematically shows a further exemplary embodiment of a modified live image;
Fig. 9 schematically shows a further exemplary embodiment of a modified live image; an
Figs 10, 11, 12, 13, 14, and 15 show photographic images of the embodiments of Figures 4, 5, 6, 7, 8, and 9 respectively.

DETAILED DESCRIPTION OF EXEMPLARY EMBODIMENTS

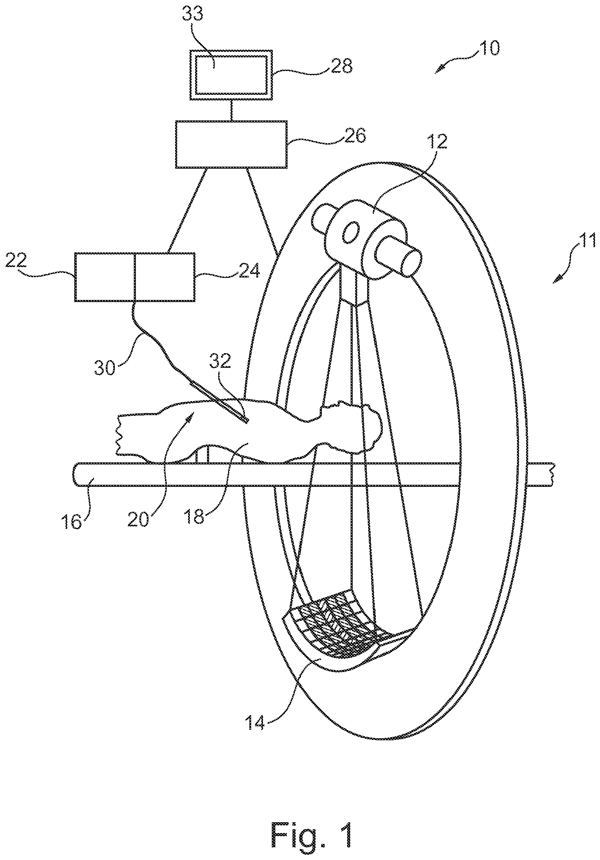

Fig. 1 shows a medical imaging apparatus 10 for providing information about an object. An image acquisition device 11 is provided which, for example, comprises a source of X-ray radiation 12 and a detector module 14. Further, a support 16 is provided, e.g. a table, to receive an object 18, for example a patient to be examined. An interventional device 20 is provided, for example a needle device. For example, the needle device is adapted for tissue inspection based on optical spectroscopy. A light source 22 and a light detector 24 are provided which are connected to a processing device 26. Further, a display device 28 is provided which is connected to the processing device 26. The processing device 26 is also connected to the image acquisition device 11 wherein the processing device 26 is capable of controlling the image acquisition device 11. The processing unit 26 is also capable of controlling the light source 22 to emit light into the interventional device 20 such that light will be emitted through the distal end into surrounding tissue. A part of the light will be reflected to the needle device, depending on the kind or type of tissue in front of the needle device 20, to be received by the light detector 24. Transmission of the light from the light source 22 to the needle and from the needle to the light detector 24 is provided by a connection 30, comprising at least two light transmitting fibers.

The light detector 24 is adapted to transform the received light reflected by the tissue into electrical signals which are then provided to the processing device 26.

The image acquisition device 11 is adapted to detect object data from at least one region of interest of the object 18. For example, the X-ray radiation source 12 emits X-ray radiation towards the detector module 14. Thus, the detector module 14 provides the electrical signals to the processing device 26 from which, for example, a fluoroscopic image can be derived.

The interventional device 20 is adapted to detect physiological parameters of the object 18 depending on the position of a predetermined area 32, for example, the distal, i.e. the front tip of the needle, of the interventional device 20 in relation to the object 18 and to provide the physiological parameters to the processing device 26.

Within the processing device 26, at least a part of the object data provided by the image acquisition device 11 is transformed into image data and the physiological parameters provided by the interventional device 20 is converted into physiological data. Further, the processing device 26 is adapted to modify at least one image parameter of the image data depending on the physiological data, thereby transforming the image data into modified live image data. The processing device 26 provides a modified live data to a display device 28 which displays a modified live image 33.

In another example, not shown, the interventional device may be a biopsy needle, a canula, a trocar, a catheter or an endoscope.

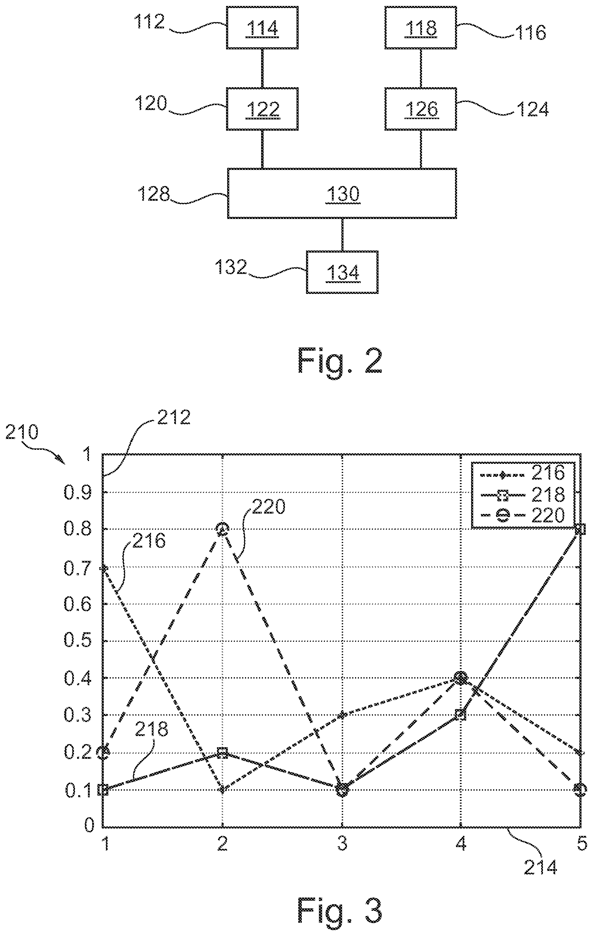

In the following, the basic method steps according to an exemplary embodiment of the invention shall be described with reference to Fig. 2. In a detection step 112, first object data 114 is detected from at least one region of interest of the object 18. In a receiving step 116, parameters 118 of the object 18 are received from the device 20 depending on the position of the predetermined location 32 of the device 20 in relation to the object 18. In a transformation step 120, at least a part of the object data 114 is transformed into image data 122. In a conversion step 124, the object parameters 118 are converted into second object data 126.

In a modification step 128, at least one image parameter of the image data 122 is modified depending on the second object data 126. Thereby, the image data 122 is transformed into modified live image data 130. Further, the modified live image data 130 is displayed 132 as a modified live image 134 on the display 28.

For example, the detection 112 of the first object data 114 can be achieved by the image acquisition device 11. The detection of the object parameter 118 can be achieved with the interventional device 20, wherein the parameters 118 may comprise physiological parameters from the patient 18. The first object data 114 and the object parameters 118 are provided to the processing device 26 where the transformation step 120 and the conversion step 124 as well as the modification step 128 are performed.

By displaying 132 the modified live image 134, the user is provided with enhanced information such that, for example, image data is provided in form of image information, meanwhile additional information, namely information in relation with the at least one object parameter, for example physiological parameters, are also provided to the user. Since the modification 128 occurs in dependency from the second object data 126, that means depending on the object parameters 118 of the object 18, the displayed image 134 is constantly changing depending on a movement or change of location of the device, for example the interventional device 20, detecting the changing object parameters 118. The first object data 114 is detected constantly, too, thus providing live first object data.

It is noted that Fig. 2 is a flow chart, showing the steps of an exemplary embodiment of a method according to the invention. It will be understood that the steps described with respect to the method, are major steps wherein these major steps might be differentiated or divided into several sub-steps. Furthermore, there might be also sub-steps between these major steps. Therefore, a sub-step is only mentioned if said step is important for the understanding of the principles of the method according to the information.

Fig. 3 shows a diagram with exemplary measurements of object parameters in form of a diagram 210 with a vertical axis 212 and a horizontal axis 214. The vertical axis 212 is related to the tissue content at the predetermined location 32 of the device 20 of the object 18. The horizontal axis 214 represents the position of the predetermined location 32 of the device 20. The tissue content is measured on a scale from 0 to 1 for each of the selected parameters. In Fig. 3, the object parameters 118 are reduced to three different parameters. For example, a first curve 216 represents the contribution of water of the tissue. A second curve 218 represents the contribution of fat. A third curve 220 represents the contribution of blood.

From the diagram 210, for each position indicated on the horizontal axis 214 with the numbers 1, 2, 3, 4 and 5, the contribution of blood, fat and water can be converted into a color code, for example, an RGB number. For example, at position 2 on axis 214, the contribution of blood, water and fat is respectively 0.8; 0.1; and 0.2. To convert this information into RGB numbers, the following formula can be used where R;G;B correspond respectively to red, green and blue (on a scale of 0 to 1).

$$R = ([blood]) / ([blood] + [water] + [fat])$$

$$G = ([fat]) / ([blood] + [water] + [fat])$$

$$B = ([water]) / ([blood] + [water] + [fat])$$

where [X] corresponds to the contribution value of element X = {blood; fat; water}.

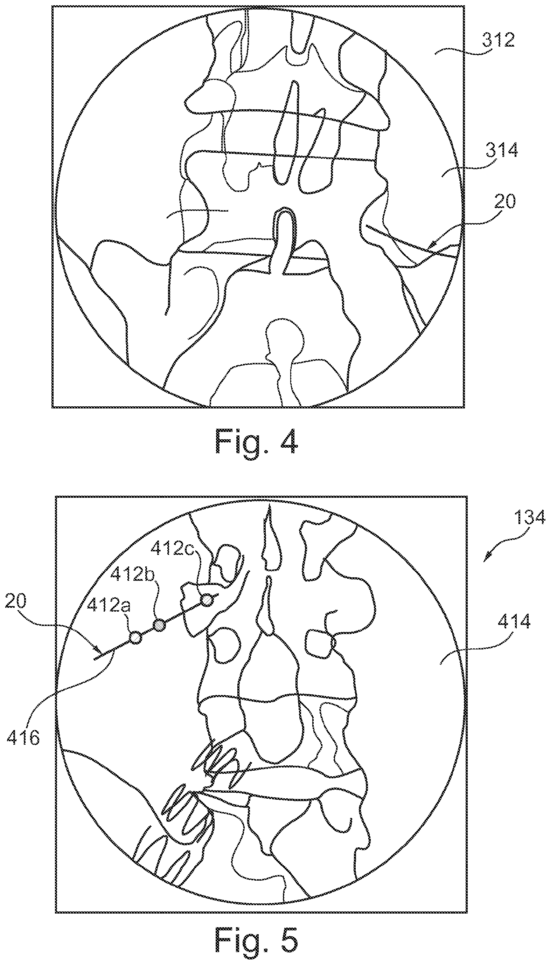

The thus derived physiological data can then be used to modify at least one image parameter of the image data depending on the physiological data. For example, the image data comprises a background 312 which is shown in Fig. 4 as surrounding parts in a rectangular display of the display device 28. The background 312 surrounds the circular fluoroscopic image 314. The fluoroscopic image 314 shows morphological structures such as structures of vertebras. Further, the device 20 is also shown in the fluoroscopic image 314. In order to provide an improved supply of information to the user which does not negatively affect the concentration of the user, the background 312 is modified with respect to at least one of its image parameters, for example, the color, depending on the physiological data. With reference to the discussion of Fig. 3, the background 312 corresponds to the optical information representing the physiological parameters, for example for position 2 on the scale 214. Thus, using the formula, mentioned above, the RGB number is RGB = (0.8; 0.2; 0.1) which is a color close to red. In other words, the background 312 is displayed in a red color.

The background color will change in real time at each different needle position while the central part 314 of the screen containing the X-ray image is not affected. In this way, the physician can proceed with the needle intervention obtaining the optical information in a way that is not compromising the time he or she can spend on interpreting the X-ray image 314.

Another exemplary embodiment is shown in Fig. 5, where instead of or even in addition to the color changing of a background, the interventional device 20 is shown in the modified live image 134 wherein the graphical representation 412 of the physiological data is shown within the fluoroscopic image 414. In case the interventional device 20 is inserted into the object 18, a trajectory 416 can be indicated by displaying the graphical representation 412 in form of colored markers 412a, 412b and 412c, such as colored circles, which can also vary in their diameters to indicate the intensity of the physiological parameter or for other purposes. Thus, a first colored marker 412a is shown in blue-like color indicating that water is contributing to the tissue at this particular location with a rather large amount. A second colored marker 412b in the color red indicates that blood is the major contributing part in that respective location within the object tissue. Further, a third colored marker 412c with a green-like color indicates a fat layer.

Using the modified live image 134, providing the spectroscopic information in addition to the fluoroscopic image, it is for example possible to avoid puncturing blood vessels during an interventional procedure. It is also possible to confirm that a fatty structure is reached by the interventional device. That fatty structure indicates the fat layer that surrounds the region of interest such as the epidural space, e.g. the spinal cord.

This makes the subject matter of the present invention suitable for minimally invasive needle interventions such as low back pain interventions. The subject matter of the invention is also suitable for taking biopsies in the field of cancer diagnosis or in cases where a tissue characterization around the needle is required.

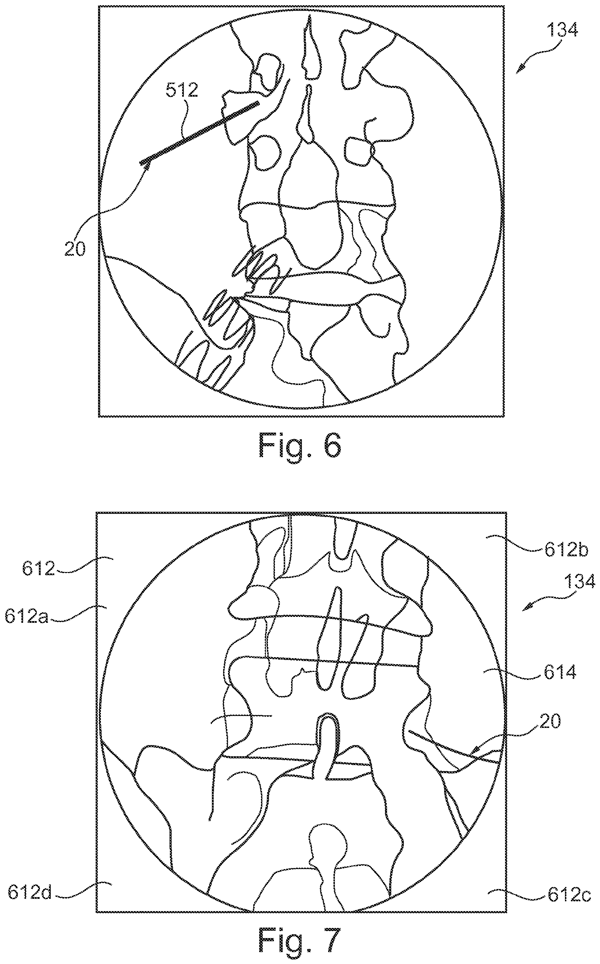

In a further exemplary embodiment shown in Fig. 6, the interventional device 20 is shown in the modified live image 134. In order to transmit additional information to the user, the interventional device 20 is shown in a colored representation 512, wherein the color of the interventional device is modified depending on the detected physiological data. Of course, the background or other parameters as well and additionally parameters may be modified providing further additional information.

Another exemplary embodiment is shown in Fig. 7. The interventional device 20 is an optical needle comprising more than two fibers such that spatial differences in the tissue to the right and left side, for instance, are detectable. For example, the interventional device 20 indicated in the fluoroscopic image 614 is capable of differentiating four different spatial directions at the tip of the needle. The four different physiological parameters are represented or visualized respectively on the display by different colors in four different parts 612a, 612b, 612c, and 612d of the background of 612. For example, in case that the situation at the tip of the needle is such that in the upper two positions of the predetermined areas of the interventional device are showing a large contribution of blood, the two corners 612a and 612b are displayed in a red color. If the lower left part at the tip of the needle is showing a large amount of water with respect to the contribution to the tissue at that location, the corner 612d is shown in a blue color. In case the tissue surrounding the lower right area of the tip of the needle is showing fat as a main part, the corner 612c is having a green-like color.

This provides the advantage that the user is provided with enhanced information indicating the expected type of tissue with respect to possible movement amendments of the interventional device 20. Of course this can be combined with the trajectory information described in relation with, for example, Fig. 5.

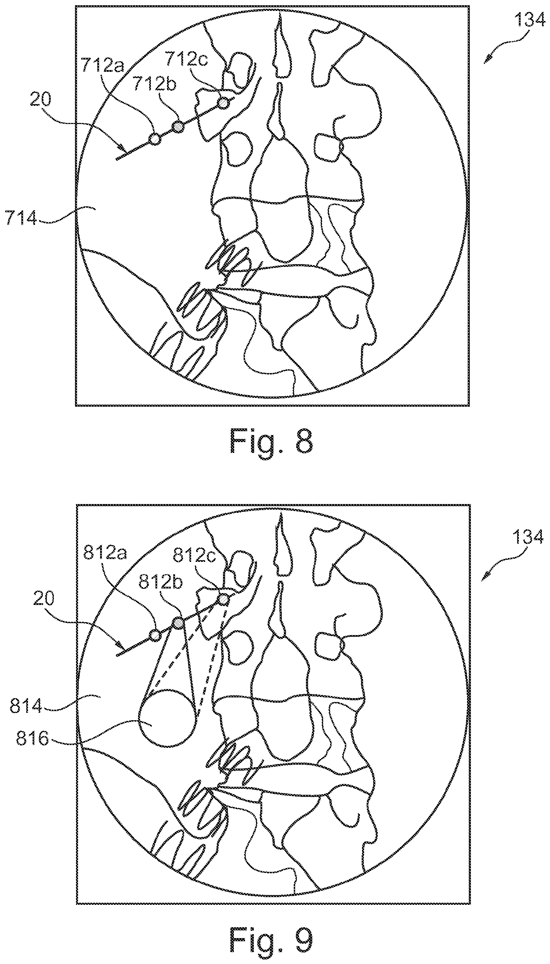

Instead of showing the RGB content described in Fig. 4 or 5, it is also possible to use three colors to provide the user with basic instructions according to predetermined values. For example, in Fig. 8 the interventional device 20 is shown with instructional markers 712 along the trajectory of the interventional device 20 within a fluoroscopic image 714. A first instructional marker 712a is shown in green as a first color indicating that no danger is expected. A second marker 712b is shown in yellow as a second color indicating that the user should be careful approaching an important structure, for example a blood vessel which the user preselected as a structure that he tries not to puncture. A third marker 712c is shown in red as a third color indicating that the tip of the interventional device 20 is very close to an important structure. Of course, this can also be combined with background colours.

By providing a modified image, it is generally possible to provide detailed information of the situation at, for example, the tip of the needle even though the fluoroscopic image may have such a resolution that it is not possible for the user to derive the information about the present situation with enough detailed information from the fluoroscopic image. In other words, a fluoroscopic image or another type of image, for example an ultrasound image, can be provided in a lower resolution representing morphological structures whereas the physiological parameter derived from the interventional device contribute to a much higher resolution providing microscopic information that is then represented in the modified image in order to improve the information supply of the user.

A further exemplary embodiment is shown in Fig. 9 where the interventional device 20 is indicated with, for example graphical markers 812 representing physiological information for the respective positions along the trajectory, as already discussed above.

In the embodiment shown in Fig. 9, a needle is used for the interventional device with which it is possible to obtain microscopic image data of the tissue in front of the needle. For example, the needle is a scanning fiber confocal microscopic needle. In addition to the modified image, for example as discussed in relation with Fig. 5, 6, 7 or 8, the obtained microscopic image data on the tip of the needle is also visualized within the fluoroscopic image 814 in a location of the fluoroscopic image 114 where a microscopic image 816 does not interfere with any structural information. For providing improved user comfort, the microscopic image 816 may be only only shown on command, which command can be given, for example, using a pedal, a mouse or a touch screen. The microscopic image 816 can be shown at the same location for different trajectory positions as shown in Fig. 9, as well as in a new location with the fluoroscopic image for each trajectory position.

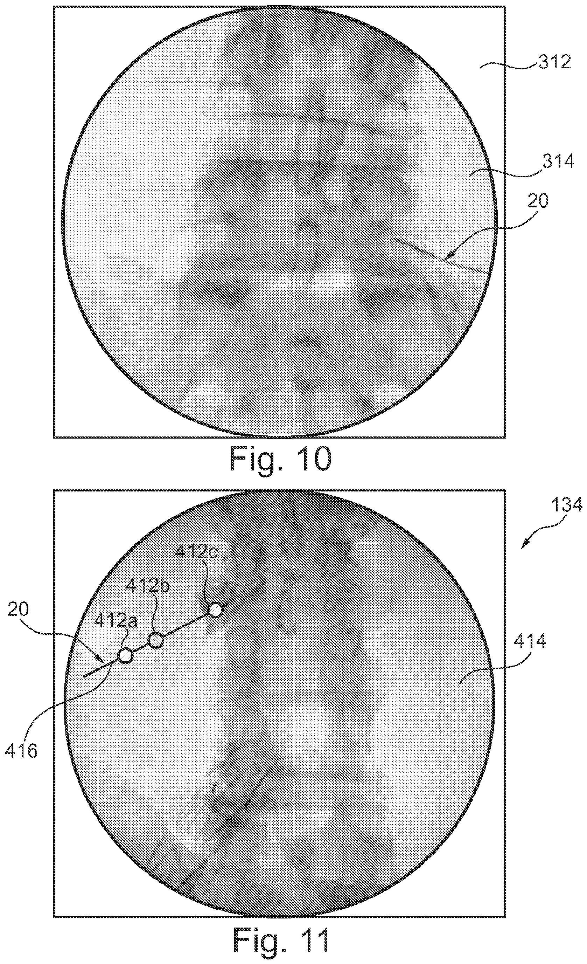

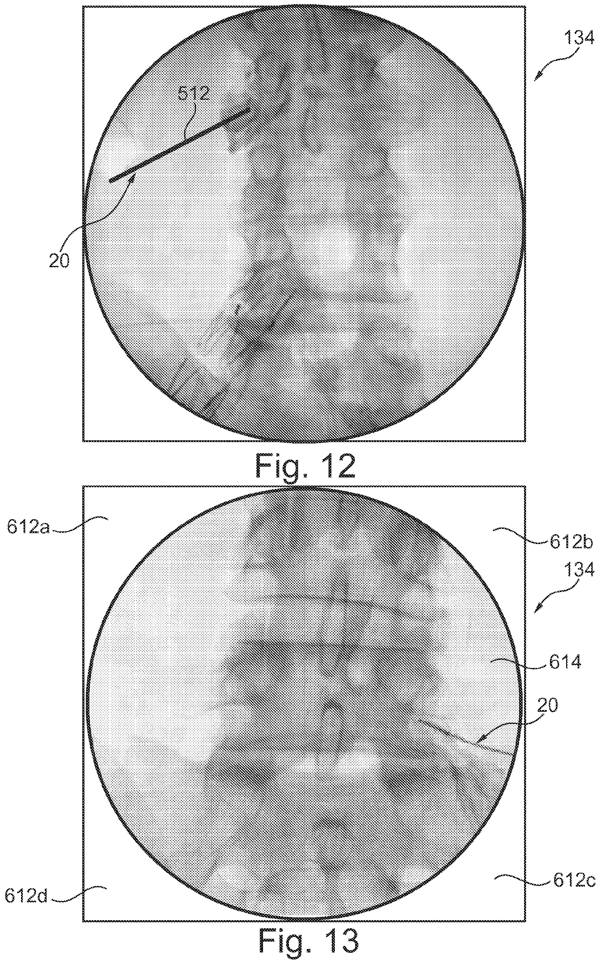

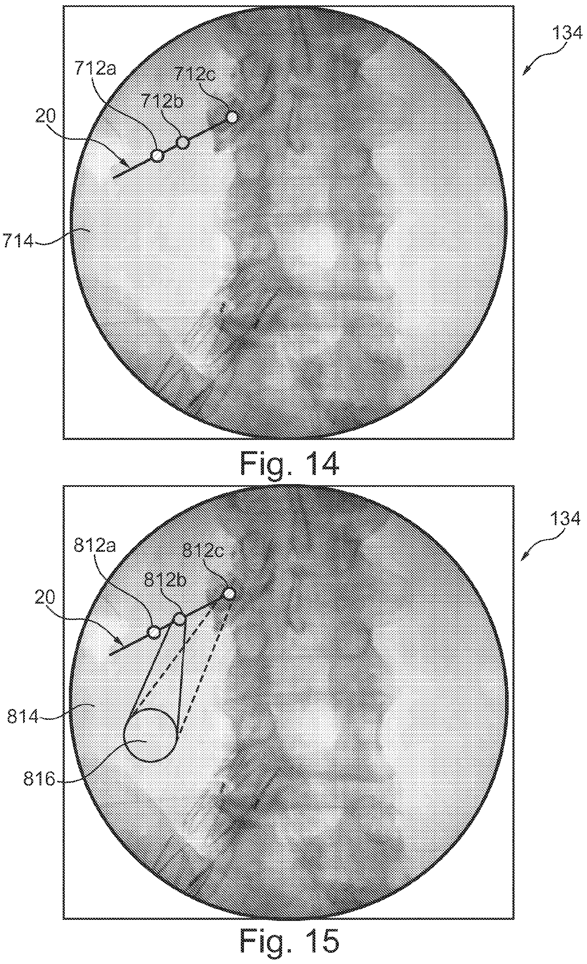

For a better understanding, figures 10 to 15 show photographic images according to the drawings of figures 4 to 9 respectively. Hence, the same reference numbers are indicated with respect to the figures 10 to 15. In order to avoid unnecessary repetition, a description with relation to figures 10 to 15 is thus not necessary.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage.

A computer program may be stored and/or distributed on a suitable medium, such as an optical storage medium or a solid state medium supplied together with or as part of other hardware, but may also be distributed in other forms, such as via the internet or other wired or wireless telecommunication systems.

Any reference signs in the claims should not be construed as limiting the scope.

## Claims

1. A medical imaging apparatus (10) for providing information about an object, comprising:

   - an image acquisition device (11);
   - an interventional device (20);
   - a processing device (26); and
   - a display device (28);

   wherein the image acquisition device (11) is adapted to detect object data (114) from at least one region of interest of an object (18) and to provide the object data (114) to the processing device (26); wherein the object data (114) is detected constantly to provide live object data (114);
   wherein the image acquisition device (11) comprises an X-ray imaging system or a computer tomography system or a magnetic resonance imaging modality or an acoustic imaging system using ultrasound waves;
   wherein the interventional device (20) is adapted to detect physiological parameters (118) of the object depending on the position of a predetermined area (32) of the interventional device (20) in relation to the object and to provide the physiological parameters (118) to the processing device (26);
   wherein the processing device (26) is adapted to transform at least a part of the live object data (114) into image data (122) and to convert the physiological parameters (118) into physiological data (126); wherein the processing device is adapted to convert the physiological parameters (118) into the physiological data (126) in real time;
   wherein the processing device (26) is adapted to modify at least one image parameter of the image data (122) depending on the physiological data (126) and to thereby transform the image data (122) into modified live image data (130); wherein the processing device (26) is adapted to to modify the at least one image parameter of the image data (122) depending on the physiological data (126) in real time;
   wherein the processing device (26) is adapted to provide the modified live image data (130) to the display device (28); and
   wherein the display device (28) is adapted to display the modified live image data (130) as a modified live image (134).

2. Apparatus according to claim 1, wherein the image data (122) comprises a background (312; 612) and wherein at least one parameter of at least a part of the background is modified in dependency of the physiological data (126).

3. Apparatus according to claim 1, wherein the interventional device (20) is shown in the modified live image (134) and wherein a graphical representation (412a, 412b, 412c) of the physiological data (126) is shown along a trajectory (416) of the interventional device (20).

4. Apparatus according to claim 1, wherein the interventional device (20) is shown in the modified live image (134) and wherein the color (512) of the interventional device (20) is modified depending on the detected physiological data (126).

5. Apparatus according to claim 1, wherein the interventional device (20) is an optical needle with at least one integrated optical fibre adapted to acquire an optical spectrum to be translated into the physiological parameters (118).

6. Apparatus according to claim 1, wherein the interventional device (20) is adapted to detect physiological parameters (118) of the object (18) depending on the position of at least two different predetermined areas of the interventional device (20) in relation to the object (18).

7. Apparatus according to claim 1, wherein the contribution of blood, fat and water in the tissue at the predetermined location of the interventional device (20) is converted into a color code; and wherein the color code is used for the modification of the image data (122).

8. Apparatus according to claim 1, wherein the processing device (26) is adapted to analyze the physiological data (126) such that the approach of an important structure is determined and the image data (122) is modified such that graphical instructional information (712a; 712b; 712c) is provided to the user.

9. Apparatus according to claim 1, wherein the interventional device (20) is adapted to acquire microscopic image data (118) of the tissue in front of the needle and to detect the physiological data (126) from the microscopic image data (118); and wherein the image data (122) is modified with at least a part of the microscopic image data (118).

10. A method for providing information about an object, comprising the following steps:

- detecting (112) first object data (114) from at least one region of interest of an object; wherein the object data (114) is detected constantly to provide live object data (114); wherein the object data (114) is detected by means of an X-ray imaging system or a computer tomography system or a magnetic resonance imaging modality or an acoustic imaging system using ultrasound waves;

- receiving (116) physiological parameters (118) of the object from an interventional device (20) depending on the position of a predetermined location of the device (20) in relation to the object;
- transforming (120) at least a part of the object data (114) into image data (122);
- converting (124) the physiological parameters (118) into physiological data (126); wherein the physiological parameters (118) are converted into the physiological data (126) in real time;
- modifying (128) at least one image parameter of the image data (122) depending on the physiological data (126) and thereby transforming the image data (122) into modified live image data (130); wherein the at least one image parameter of the image data (122) is modified depending on the physiological data (126) in real time; and
- displaying (132) the modified live image data (130) as a modified live image (134).

11. Computer program element for controlling an apparatus according to claim 1, which, when being executed by a processing unit, is adapted to perform the method steps of claim 10.

12. Computer readable medium having stored the program element of claim 11.


**Patentansprüche**

1. Medizinisches Bildgebungsgerät (10) zur Bereitstellung von Informationen über ein Objekt, umfassend:

- eine Bilderfassungsvorrichtung (11);
- eine interventionelle Vorrichtung (20);
- eine Verarbeitungsvorrichtung (26); und
- eine Anzeigevorrichtung (28);

wobei die Bilderfassungsvorrichtung (11) dafür ausgelegt ist, Objektdaten (114) aus mindestens einer interessierenden Region eines Objekts (18) zu detektieren und die Objektdaten (114) der Verarbeitungsvorrichtung (26) bereitzustellen; wobei die Objektdaten (114) fortwährend detektiert werden, um Live-Objektdaten (114) bereitzustellen; wobei die Bilderfassungsvorrichtung (11) ein Röntgenbildgebungssystem oder ein Computertomographiesystem oder eine Magnetresonanzbildgebungsmodalität oder ein akustisches Bildgebungssystem mit Ultraschallwellen umfasst; wobei die interventionelle Vorrichtung (20) dafür ausgelegt ist, physiologische Parameter (118) des Objekts abhängig von der Position eines vorgegebenen Bereichs (32) der interventionellen Vorrichtung (20) in Beziehung auf das Objekt zu detektieren und die physiologischen Parameter (118) der Verarbeitungsvorrichtung (26) bereitzustellen;

wobei die Verarbeitungsvorrichtung (26) dafür ausgelegt ist, mindestens einen Teil der Live-Objektdaten (114) in Bilddaten (122) zu transformieren und die physiologischen Parameter (118) in physiologische Daten (126) umzuwandeln; wobei die Verarbeitungsvorrichtung dafür ausgelegt ist, die physiologischen Parameter (118) in Echtzeit in die physiologischen Daten (126) umzuwandeln;

wobei die Verarbeitungsvorrichtung (26) dafür ausgelegt ist, mindestens einen Bildparameter der Bilddaten (122) abhängig von den physiologischen Daten (126) zu modifizieren und dadurch die Bilddaten (122) in modifizierte Live-Bilddaten (130) zu transformieren; wobei die Verarbeitungsvorrichtung (26) dafür ausgelegt ist, den mindestens einen Bildparameter der Bilddaten (122) abhängig von den physiologischen Daten (126) in Echtzeit zu modifizieren;

wobei die Verarbeitungsvorrichtung (26) dafür ausgelegt ist, die modifizierten Live-Bilddaten (130) der Anzeigevorrichtung (28) bereitzustellen; und

wobei die Anzeigevorrichtung (28) dafür ausgelegt ist, die modifizierten Live-Bilddaten (130) als ein modifiziertes Live-Bild (134) anzuzeigen.

2. Gerät nach Anspruch 1, wobei die Bilddaten (122) einen Hintergrund (312; 612) umfassen und wobei der mindestens eine Parameter von mindestens einem Teil des Hintergrunds in Abhängigkeit von den physiologischen Daten (126) modifiziert wird.

3. Gerät nach Anspruch 1, wobei die interventionelle Vorrichtung (20) in dem modifizierten Live-Bild (134) gezeigt wird und wobei die grafische Darstellung (412a, 412b, 412c) der physiologischen Daten (126) entlang einer Trajektorie (416) der interventionellen Vorrichtung (20) gezeigt wird.

4. Gerät nach Anspruch 1, wobei die interventionelle Vorrichtung (20) in dem modifizierten Live-Bild (134) gezeigt wird und wobei die Farbe (512) der interventionellen Vorrichtung (20) abhängig von den detektierten physiologischen Daten (126) modifiziert wird.

5. Gerät nach Anspruch 1, wobei die interventionelle Vorrichtung (20) eine optische Kanüle mit mindestens einer integrierten optischen Faser ist, die dafür ausgelegt ist, ein optisches Spektrum zu erfassen, das in die physiologischen Parameter (118) umzusetzen ist.

6. Gerät nach Anspruch 1, wobei die interventionelle Vorrichtung (20) dafür ausgelegt ist, physiologische Parameter (118) des Objekts (18) abhängig von der Position von mindestens zwei verschiedenen vorgegebenen Bereichen der interventionellen Vorrichtung (20) in Bezug auf das Objekt (18) zu detektieren.

7. Gerät nach Anspruch 1, wobei der Beitrag von Blut, Fett und Wasser in dem Gewebe an dem vorgegebenen Ort der interventionellen Vorrichtung (20) in einen Farbcode umgewandelt wird; und wobei der Farbcode für die Modifizierung der Bilddaten (122) verwendet wird.

8. Gerät nach Anspruch 1, wobei die Verarbeitungsvorrichtung (26) dafür ausgelegt ist, die physiologischen Daten (126) derartig zu analysieren, dass die Annäherung einer wichtigen Struktur ermittelt wird und die Bilddaten (122) derartig modifiziert werden, dass grafische Anweisungsinformationen (712a; 712b; 712c) für den Benutzer bereitgestellt werden.

9. Gerät nach Anspruch 1, wobei die interventionelle Vorrichtung (20) dafür ausgelegt ist, mikroskopische Bilddaten (118) des Gewebes vor der Kanüle zu erfassen und die physiologischen Daten (126) aus den mikroskopischen Bilddaten (118) zu detektieren; und wobei die Bilddaten (122) mit mindestens einem Teil der mikroskopischen Bilddaten (118) modifiziert werden.

10. Verfahren zum Bereitstellen von Informationen über ein Objekt, wobei das Verfahren die folgenden Schritte umfasst:

- Detektieren (112) erster Objektdaten (114) aus mindestens einer interessierenden Region eines Objekts;

wobei die Objektdaten (114) fortwährend detektiert werden, um Live-Objektdaten (114) bereitzustellen;

wobei die Objektdaten (114) mittels eines Röntgenbildgebungssystems oder eines Computertomographiesystems oder einer Magnetresonanzbildgebungsmodalität oder eines akustischen Bildgebungssystem mit Ultraschallwellen detektiert werden;

- Empfangen (116) physiologischer Parameter (118) des Objekts von einer interventionellen Vorrichtung (20) abhängig von der Position eines vorgegebenen Orts der Vorrichtung (20) in Bezug auf das Objekt;

- Transformieren (120) von mindestens einem Teil der Objektdaten (114) in Bilddaten (122);

- Umwandeln (124) der physiologischen Parameter (118) in physiologische Daten (126);

wobei die physiologischen Parameter (118) in Echtzeit in die physiologischen Daten (126) umgewandelt werden;

- Modifizieren (128) von mindestens einem Bildparameter der Bilddaten (122) abhängig von den physiologischen Daten (126) und dadurch Transformieren der Bilddaten (122) in modifizierte Live-Bilddaten (130);

wobei der mindestens eine Bildparameter der Bilddaten (122) abhängig von den physiologischen Daten (126) in Echtzeit modifiziert wird; und

- Anzeigen (132) der modifizierten Live-Bilddaten (130) als ein modifiziertes Live-Bild (134).

**11.** Computerprogrammelement zum Steuern eines Geräts nach Anspruch 1, das, wenn es durch eine Verarbeitungseinheit ausgeführt wird, dafür ausgelegt ist, die Verfahrensschritte nach Anspruch 10 durchzuführen.

**12.** Computerlesbares Medium, auf dem das Programmelement nach Anspruch 11 gespeichert ist.

**Revendications**

**1.** Appareil d'imagerie médicale (10) pour fournir des informations sur un objet, comprenant :

- un dispositif d'acquisition d'images (11) ;
- un dispositif interventionnel (20) ;
- un dispositif de traitement (26) ; et
- un dispositif d'affichage (28) ;

dans lequel le dispositif d'acquisition d'images (11) est adapté pour détecter des données d'objet (114) à partir d'au moins une région d'intérêt d'un objet (18) et pour fournir les données d'objet (114) au dispositif de traitement (26) ; dans lequel les données d'objet (114) sont détectées de manière constante pour fournir des données d'objet en direct (114) ; dans lequel le dispositif d'acquisition d'images (11) comprend un système d'imagerie par rayons X ou un système de tomodensitométrie ou une modalité d'imagerie par résonnance magnétique ou un système d'imagerie acoustique utilisant des ondes ultrasonores ;

dans lequel le dispositif interventionnel (20) est adapté pour détecter les paramètres physiologiques (118) de l'objet en fonction de la position d'une zone prédéterminée (32) du dispositif interventionnel (20) par rapport à l'objet et pour fournir les paramètres physiologiques (118) au dispositif de traitement (26) ;

dans lequel le dispositif de traitement (26) est adapté pour transformer au moins une partie des données d'objet en direct (114) en des données d'image (122) et pour convertir les paramètres physiologiques (118) en données physiologiques (126) ; dans lequel le dispositif de traitement est adapté pour convertir les paramètres physiologiques (118) en les données physiologiques (126) en temps réel ;

dans lequel le dispositif de traitement (26) est adapté pour modifier au moins un paramètre d'image des données d'image (122) en fonction des données physiologiques (126) et pour transformer ainsi les données d'image (122) en données d'image en direct modifiées (130) ; dans lequel le dispositif de traitement (26) est adapté pour modifier l'au moins un paramètre d'image des données d'image (122) en fonction des données physiologiques (126) en temps réel ;

dans lequel le dispositif de traitement (26) est adapté pour fournir les données d'image en direct modifiées (130) au dispositif d'affichage (28) ; et

dans lequel le dispositif d'affichage (28) est adapté pour afficher les données d'image en direct modifiées (130) sous la forme d'une image en direct modifiée (134).

**2.** Appareil selon la revendication 1, dans lequel les données d'image (122) comprennent un arrière-plan (312 ; 612) et dans lequel au moins un paramètre d'au moins une partie de l'arrière-plan est modifié en fonction des données physiologiques (126).

**3.** Appareil selon la revendication 1, dans lequel le dispositif interventionnel (20) est représenté dans l'image en direct modifiée (134) et dans lequel une représentation graphique (412a, 412b, 412c) des données physiologiques (126) est représentée le long d'une trajectoire (416) du dispositif interventionnel (20).

**4.** Appareil selon la revendication 1, dans lequel le dispositif interventionnel (20) est représenté dans l'image en direct modifiée (134) et dans lequel la couleur (512) du dispositif interventionnel (20) est modifiée en fonction des données physiologiques détectées (126).

**5.** Appareil selon la revendication 1, dans lequel le dispositif interventionnel (20) est une aiguille optique avec au moins une fibre optique intégrée adaptée pour acquérir un spectre optique devant être traduit en les paramètres physiologiques (118).

**6.** Appareil selon la revendication 1, dans lequel le dispositif interventionnel (20) est adapté pour détecter les paramètres physiologiques (118) de l'objet (18) en fonction de la position d'au moins deux zones prédéterminées différentes du dispositif interventionnel (20) par rapport à l'objet (18).

**7.** Appareil selon la revendication 1, dans lequel la contribution du sang, de la matière grasse et de l'eau dans le tissu à l'emplacement prédéterminé du dispositif interventionnel (20) est convertie en un code couleur ; et dans lequel le code couleur est utilisé pour la modification des données d'image (122).

**8.** Appareil selon la revendication 1, dans lequel le dispositif de traitement (26) est adapté pour analyser les données physiologiques (126) de telle sorte que l'approche d'une structure importante est déterminée et les données d'image (122) sont modifiées de telle sorte que des informations instructionnelles graphiques (712a ; 712b ; 712c) sont fournies à l'utilisateur.

9. Appareil selon la revendication 1, dans lequel le dispositif interventionnel (20) est adapté pour acquérir des données d'image microscopiques (118) du tissu en face de l'aiguille et pour détecter les données physiologiques (126) à partir des données d'image microscopiques (118) ; et dans lequel les données d'image (122) sont modifiées avec au moins une partie des données d'image microscopiques (118).

10. Procédé de fourniture d'informations sur un objet, comprenant les étapes suivantes :

   - la détection (112) de premières données d'objet (114) à partir d'au moins une région d'intérêt d'un objet ;
   - dans lequel les données d'objet (114) sont détectées de manière constante pour fournir des données d'objet en direct (114) ;
   dans lequel les données d'objet (114) sont détectées au moyen d'un système d'imagerie par rayons X ou d'un système de tomodensitométrie ou d'une modalité d'imagerie par résonnance magnétique ou d'un système d'imagerie acoustique utilisant des ondes ultrasonores ;
   - la réception (116) de paramètres physiologiques (118) de l'objet à partir d'un dispositif interventionnel (20) en fonction de la position d'un emplacement prédéterminé du dispositif interventionnel (20) par rapport à l'objet ;
   - la transformation (120) d'au moins une partie des données d'objet (114) en données d'image (122) ;
   - la conversion (124) des paramètres physiologiques (118) en données physiologiques (126) ;
   - dans lequel les paramètres physiologiques (118) sont convertis en les données physiologiques (126) en temps réel ;
   - la modification (128) d'au moins un paramètre d'image des données d'image (122) en fonction des données physiologiques (126) et ainsi, la transformation des données d'image (122) en données d'image en direct modifiées (130) ;
   dans lequel l'au moins un paramètre d'image des données d'image (122) est modifié en fonction des données physiologiques (126) en temps réel ; et
   - l'affichage (132) des données d'image en direct modifiées (130) sous la forme d'une image en direct modifiée (134).

11. Elément de programme informatique pour commander un appareil selon la revendication 1, qui, lorsqu'il est exécuté par une unité de traitement, est adapté pour effectuer les étapes de procédé selon la revendication 10.

12. Support lisible par ordinateur stockant l'élément de programme selon la revendication il.

Fig. 1

Fig. 2

Fig. 3

Fig. 4

Fig. 5

**Fig. 6**

**Fig. 7**

Fig. 8

Fig. 9

Fig. 10

Fig. 11

**Fig. 12**

**Fig. 13**

Fig. 14

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070016028 A1

- EP 1415608 A2